(19) Europäisches Patentamt
European Patent Office
Office européen des brevets

(11) Veröffentlichungsnummer: **0 201 030**
**B1**

(12) **EUROPÄISCHE PATENTSCHRIFT**

(45) Veröffentlichungstag der Patentschrift: **12.09.90**

(51) Int. Cl.$^5$: **C 07 D 251/34,** C 07 C 323/20, C 07 C 275/62

(21) Anmeldenummer: **86105901.2**

(22) Anmeldetag: **29.04.86**

(54) **Verfahren zur Herstellung von 1,3,5-Triazintrionen.**

(30) Priorität: **09.05.85 DE 3516632**

(43) Veröffentlichungstag der Anmeldung:
**12.11.86 Patentblatt 86/46**

(45) Bekanntmachung des Hinweises auf die Patenterteilung:
**12.09.90 Patentblatt 90/37**

(84) Benannte Vertragsstaaten:
**BE CH DE FR GB IT LI NL SE**

(56) Entgegenhaltungen:
**DE-A-2 115 096**

**JOURNAL OF THE AMERICAN CHEMICAL SOCIETY, Band 75, Nr. 15, 5. August 1953, Seiten 3615-3618, The American Chemical Society; W.J. CLOSE: "Anticonvulsant drugs. VII. Some monosubstituted isocyanurates"**

**CHEMICAL ABSTRACTS, Band 100, Nr. 13, 26. März 1984, Seite 614, Zusammenfassung Nr. 102973v, Columbus, Ohio, US; & JP-A-152 859 (TAIHO PHARMACEUTICAL CO.) 10-09-1983**

(73) Patentinhaber: **BAYER AG**
**D-5090 Leverkusen 1 Bayerwerk (DE)**

(72) Erfinder: **Gallenkamp, Bernd, Dr.**
**Claudiusweg 5**
**D-5600 Wuppertal 1 (DE)**
Erfinder: **Günther, Andreas, Dr.**
**Belaweg 12**
**D-5000 Köln 80 (DE)**
Erfinder: **Mohrmann, Karl-Heinrich, Dr.**
**Waldstrasse 9**
**D-5060 Bergisch-Gladbach 2 (DE)**
Erfinder: **Schmidt, Thomas, Dr.**
**Ginsterweg 9**
**D-5657 Haan 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 201 030 B1

**Beschreibung**

Die Erfindung betrifft neue Biurete, ihre Herstellung und ein neues Verfahren zur Herstellung von 1,3,5-Triazintrionen.

Es ist bereits bekannt, daß man 1,3,5-Triazintrione aus Harnstoffen und Chlorcarbonylisocyanaten herstellen kann (=DE—OS 2 413 722; DE—OS 2 718 799).

Nachteilig bei diesem Verfahren ist der Einsatz von Carbonylisocyanaten. Die Herstellung und Handhabung dieser Verbindungen im großtechnischen Maßstab sind problematisch. Auch für die Lagerung dieser Zwischenprodukte sind aufwendige Sicherheitsmaßnahmen notwendig.

Die Bildung von Triazinonen aus Phenyl- bzw. Benzyl- substituierten Biureten ist bekannt durch N. J. Close et al, J.A.C.S 75 (1953), S. 3618. Die dort beschriebenen Ausbeuten sind jedoch nicht in jedem Fall befriedigend.

Aus DE—OS 2 115 096 ist N-4-(4-chlorphenoxy)-phenyl-N'-methyl-N'-chlorcarbonylharnstoff bekannt. Es geht daraus jedoch nichts über seine Verwendung zur weiteren Umsetzung zu Triazintrionen hervor.

Die vorliegende Erfindung betrifft:

Biurete der Formel (II)

$$R—NH—CO—NR^1—CO—NHR^2 \quad (II)$$

in welcher

R für einen Rest

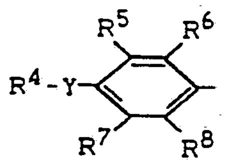

steht, wobei

$R^4$ für gegebenenfalls durch Halogen, wie Fluor, Chlor oder Brom, Cyano, Nitro, Amino, $C_1$—$C_4$-Alkyl, $C_1$—$C_4$-Alkoxy, $C_1$—$C_4$-Alkylthio, $C_1$—$C_4$-Alkylsulfinyl, $C_1$—$C_4$-Alkylsulfonyl, Halogen-$C_1$—$C_4$-Alkyl, Halogen-$C_1$—$C_4$-Alkoxy, Halogen-$C_1$—$C_4$-Alkylthio, Halogen-$C_1$—$C_4$-Alkylsulfonyl, Hydroxycarbonyl, $C_1$—$C_4$-Alkylcarbonyl, $C_1$—$C_4$-Alkoxycarbonyl, Aminocarbonyl, $C_1$—$C_4$-Alkoxycarbonylamino und/oder Sulfonylamino substituiertes Phenyl steht oder für den Rest

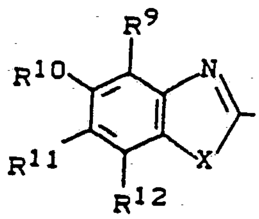

steht, wobei

$R^9$, $R^{10}$, $R^{11}$ und $R^{12}$ gleich oder verschieden sind und für Wasserstoff, Halogen wie Fluor, Chlor oder Brom, $C_1$—$C_4$-Alkyl, $C_1$—$C_4$-Alkyl, $C_1$—$C_4$-Alkoxy, Halogen-$C_1$—$C_4$-Alkyl/oder Halogen-$C_1$—$C_4$-Alkoxy stehen und

X für Sauerstoff, Schwefel oder eine Gruppierung —N=CH— steht,

$R^5$, $R^6$, $R^7$ und $R^8$ gleich oder verschieden sind und für Wasserstoff, Halogen, wie Fluor, Chlor oder Brom, $C_1$—$C_4$-Alkyl oder $C_1$—$C_4$-Alkoxy stehen und

Y für Sauerstoff, Schwefel oder die Gruppierungen —CO—, —SO—, $SO_2$—, —$CH_2$—, —$CH_2O$— oder —$OCH_2$— steht und

$R^1$ und $R^2$ für Wasserstoff, Halogen, für gegebenenfalls durch Halogen substituiertes $C_1$—$C_6$-Alkyl, $C_1$—$C_6$-Alkoxy, $C_1$—$C_6$-Alkylthio und $C_1$—$C_6$-Alkylsulfonyl, sowie für gegebenenfalls durch Halogen, $C_1$—$C_2$-Alkyl, $C_1$—$C_2$-Alkoxy, $C_1$—$C_2$-Alkylthio, Halogen-$C_1$—$C_2$-Alkyl, Halogen-$C_1$—$C_2$-Alkoxy und/oder Halogen-$C_1$—$C_2$-Alkylthio substituiertes Phenyl stehen.

Verfahren zur Herstellung der Biurete der Formel (II),

$$R—NH—CO—NR^1—CO—NHR^2 \quad (II)$$

in welcher

R für einen Rest

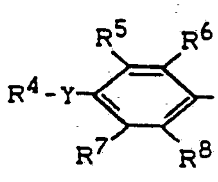

steht, wobei

$R^4$ für gegebenenfalls durch Halogen, wie Fluor, Chlor oder Brom, Cyano, Nitro, Amino, $C_1$—$C_4$-Alkyl, $C_1$—$C_4$-Alkoxy, $C_1$—$C_4$-Alkylthio, $C_1$—$C_4$-Alkylsulfinyl, $C_1$—$C_4$-Alkylsulfonyl, Halogen-$C_1$—$C_4$-Alkyl, Halogen-$C_1$—$C_4$-Alkoxy, Halogen-$C_1$—$C_4$-Alkylthio, Halogen-$C_1$—$C_4$-Alkylsulfonyl, Hydroxycarbonyl, $C_1$—$C_4$-Alkylcarbonyl, $C_1$—$C_4$-Alkoxycarbonyl, Aminocarbonyl, $C_1$—$C_4$-Alkoxycarbonylamino und/oder Sulfonylamino substituiertes Phenyl steht oder für den Rest

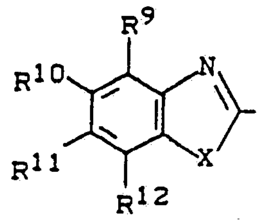

steht, wobei

$R^9$, $R^{10}$, $R^{11}$ und $R^{12}$ gleich oder verschieden sind und für Wasserstoff, Halogen wie Fluor, Chlor oder Brom, $C_1$—$C_4$-Alkyl, $C_1$—$C_4$-Alkyl, $C_1$—$C_4$-Alkoxy, Halogen-$C_1$—$C_4$-Alkyl/oder Halogen-$C_1$—$C_4$-Alkoxy stehen und

X für Sauerstoff, Schwefel oder eine Gruppierung —N=CH— steht,

$R^5$, $R^6$, $R^7$ und $R^8$ gleich oder verschieden sind und für Wasserstoff, Halogen, wie Fluor, Chlor oder Brom, $C_1$—$C_4$-Alkyl oder $C_1$—$C_4$-Alkoxy stehen und

Y für Sauerstoff, Schwefel oder die Gruppierungen —CO—, —SO—, $SO_2$—, —$CH_2$—, —$CH_2O$— oder —$OCH_2$— steht und

$R^1$ und $R^2$ für Wasserstoff, Halogen, für gegebenenfalls durch Halogen substituiertes $C_1$—$C_6$-Alkyl, $C_1$—$C_6$-Alkoxy, $C_1$—$C_6$-Alkylthio und $C_1$—$C_6$-Alkylsulfonyl, sowie für gegebenenfalls durch Halogen, $C_1$—$C_2$-Alkyl, $C_1$—$C_2$-Alkoxy, $C_1$—$C_2$-Alkylthio, Halogen-$C_1$—$C_2$-Alkyl, Halogen-$C_1$—$C_2$-Alkoxy und/oder Halogen-$C_1$—$C_2$-Alkylthio substituiertes Phenyl stehen

das dadurch gekennzeichnet ist, daß man

a) Isocyanate der Formel (IV)

$$R—NCO \quad (IV)$$

in welcher R die oben angegebene Bedeutung hat, mit Harnstoffen der Formel (V)

$$R^1NH—CO—NHR^2 \quad (V)$$

in welcher $R^1$ und $R^2$ die angegebenen Bedeutungen haben, gegebenenfalls in Gegenwart von Verdünnungsmitteln umsetzt, oder

b) für den Fall, daß Verbindungen der Formel II hergestellt werden in denen

$R^2$ für Wasserstoff steht, dadurch gekennzeichnet, daß man in der 1. Stufe Amine der Formel (VI),

$$R—NH_2 \quad (VI)$$

in welcher R die oben angegebenen Bedeutungen haben, mit Verbindungen der Formel (VII)

$$R^1NCO \quad (VII)$$

in welcher $R^1$ die oben angegebenen Bedeutungen hat, in Gegenwart von inerten Verdünnungsmitteln bei Temperaturen zwischen 0°C und 100°C zu den Verbindungen der Formel (VIII)

$$R—NH—CO—NR^1H \quad (VIII)$$

in welcher R und $R^1$ die oben angegebenen Bedeutungen haben, umsetzt und anschließend gegebenenfalls nach ihrer Isolierung, in einer 2. Stufe mit Phosgen, gegebenenfalls in Gegenwart von inerten Verdünnungsmitteln bei Temperaturen zwischen 0°C und 100°C zu den N-Chlorcarbonylharnstoffen der Formel (IX)

$$R—NH—CO—NR^1—COCl \quad (IX)$$

in welcher R und $R^1$ die oben angegebenen Bedeutungen haben, umsetzt und anschließend gegebenenfalls nach ihrer Isolierung in einer 3. Stufe, mit Ammoniak, gegebenenfalls in Gegenwart von inerten Verdünnungsmitteln bei Temperaturen zwischen 0°C und 100°C umsetzt.

Verwendung der Biurete der Formel II gemäß 1 (oben) zur Herstellung von 1,3,5-Triazintrionen der Formel I

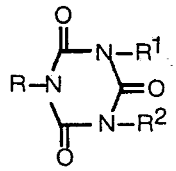

I

in welcher R, $R^1$, $R^2$ die bei 1 (oben) angegebene Bedeutung haben, dadurch gekennzeichnet, daß man die Biurete der Formel II mit Kohlensäuredialkylestern der Formel III

$$(R^3O)_2CO \qquad III$$

in welcher $R^3$ für $C_1$—$C_4$-Alkyl steht, in Gegenwart von starken Basen, gegebenenfalls in Gegenwart von Verdünnungsmitteln umsetzt.

N-Chlorcarbonylharnstoffe der Formel (IX)

$$R—NH—CO—NR^1—COCl \qquad (IX)$$

in welcher die Reste R und $R^1$ die gemäß 1 (oben) angegebene Bedeutung besitzen, ausgenommen N-4(4-chlorphenoxy)-phenyl-N'-methyl-N'-chlorcarbonylharnstoff.

Verfahren zur Herstellung der N-Chlorcarbonylharnstoffe der Formel IX gemäß 5 (oben), dadurch gekennzeichnet, daß man Verbindungen der Formel VIII

$$R—NH—CO—NR^1H \qquad (VIII)$$

in welcher R und $R^1$ die gemäß 5 (oben) angegebene Bedeutung haben, mit Phosgen umsetzt.

Überraschenderweise lassen sich mit Hilfe der erfindungsbemäßen Biurete der Formel (II) die Triazintrione der Formel I in guter Reinheit und mit hohen Ausbeuten herstellen.

Vorteilhaft bei diesem Verfahren ist, daß die einzusetzenden Biurete auch im großtechnischen Maßstab verhältnismäßig einfach hergestellt werden können. Weiterhin sind für die Lagerung der Zwischenprodukte keine aufwendige Sicherheitsmaßnahmen notwendig.

Mit Hilfe der erfindungsgemäßen Biurete der Formel II werden vorzugsweise diejenigen Verbindungen der Formel (I) hergestellt, in welcher

R die oben angegebenen Bedeutungen hat,

$R^1$ und $R^2$ für Wasserstoff, Fluor, Chlor, Brom oder für gegebenenfalls durch Fluor, Chlor und/oder Brom substituiertes $C_1$—$C_4$-Alkyl, $C_1$—$C_4$-Alkoxy, $C_1$—$C_4$-Alkylthio und $C_1$—$C_4$-Alkylsulfonyl sowie für gegebenenfalls durch Fluor, Chlor, Brom, Methyl, Methoxy, Methylthio, Trifluormethyl, Trifluormethoxy und/oder Trifluormethylthio substituiertes Phenyl stehen, wobei $R^1$ und $R^2$ nicht gleichzeitig für Wasserstoff stehen.

Im einzelnen seien folgende Verbindungen der Formel I genannt:

1-[4-(4-Phenoxy)-phenyl]-, 1-[3,5-Dichlor-4-(4-Cyano-phenoxy)-phenyl]-, 1-[3,5-Dichlor-4-(4-trifluormethylsulfonyl-phenoxy)-phenyl-, 1-[3,5-Dimethyl-4-(4-trifluormethylsulfonyl-phenoxy)-phenyl]-, 1-[3-Chlor-4-(4-trifluormethylthio-phenoxy)-phenyl]-, 1-[3-Methoxy-4-(4-trifluormethylthio-phenoxy)-phenyl]-, 1-[2-Chlor-4-(4-trifluormethylthio-phenoxy)-phenyl]- und 1-[3-Methyl-4-(5,6-dichlor-2-benzthiazolyloxy)-phenyl]-3-methyl-1,3,5-triazin-2,4,6-trion.

Verwendet man beispielsweise für die Herstellung der Verbindungen der Formel I (Verfahren 4) als Ausgangsstoffe Kohlensäurediethylester und 1-Methyl-5-[3-methyl-4-(4-trifluormethylthio-phenoxy)-phenyl]-biuret und Kalium-tert.-butylat als Säureakzeptor bzw. 3-Methyl-5-[3-methyl-4-(4-trifluormethylthiophenoxy)-phenyl]-biuret und Kohlensäurediethylester als Ausgangsstoffe und Natriummethylat als Säureakzeptor, so kann die Reaktion durch die folgenden Formelschemata wiedergegeben werden:

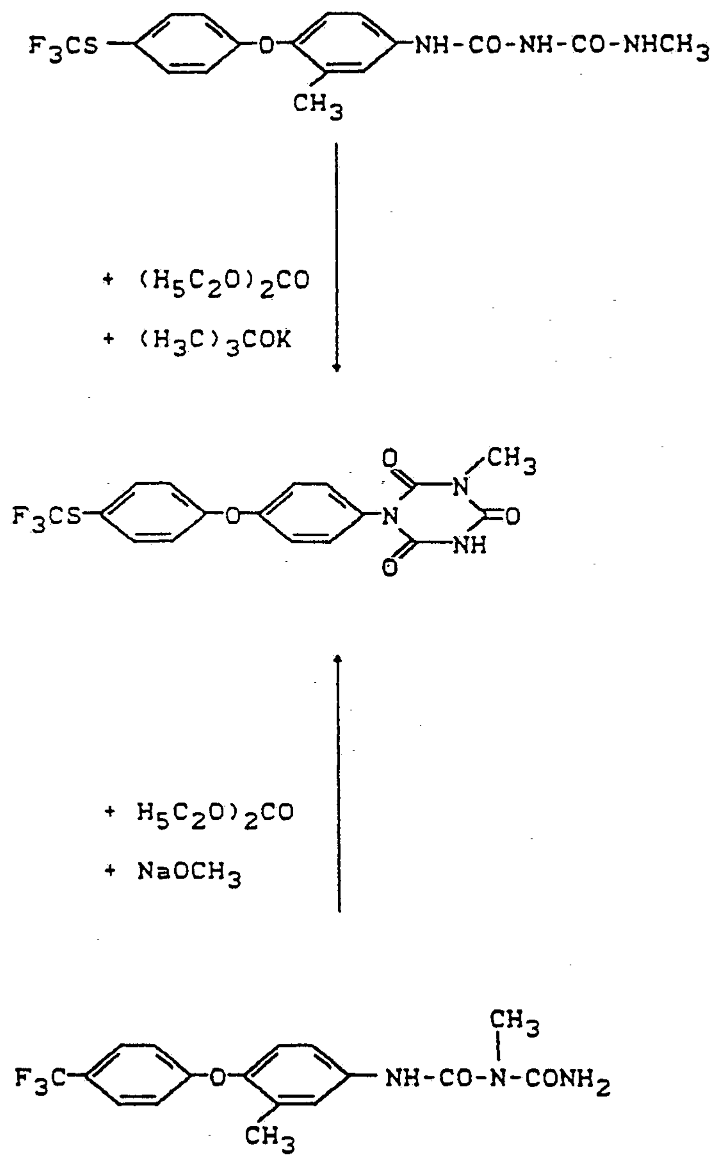


Als Ausgangsstoffe für das erfindungsgemäße Verfahren zur Herstellung der Verbindungen der Formel I seien folgende Verbindungen der Formel II genannt:

1-Methyl-5-[4-(4-phenoxy)-phenyl]-, -5-[3,5-dichlor-4-(4-cyano-phenoxy)-phenyl]-, -5-[3,5-dichlor-4-(4-trifluormethylsulfonyl-phenoxy)-phenyl]-, -5-[3,5-dimethyl-4-(4-trifluormethyl-sulfonyl-phenoxy)-phenyl]-, -5-[3-chlor-4-(4-trifluormethylthio-phenoxy)-phenyl]-, -5-[3-methoxy-4-(4-trifluormethylthio-phenoxy)-phenyl]-, -5-[2-Chlor-4-(4-trifluormethylthio-phenoxy)-phenyl]- und -5-[3-methyl-4-(5,6-dichlor-2-benz-thiazolyloxy)-phenyl]-biuret und die entsprechenden 3-Methyl-Derivate dieser Verbindungen.

Die Verbindungen der Formel (II) sind neu. Bevorzugt sind die neuen Verbindungen der Formel (II), in welcher

R für einen Rest

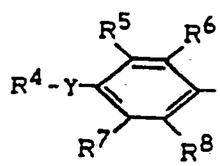

steht, wobei

$R^4$ für einen gegebenenfalls durch Fluor, Chlor, Brom, Cyano, Nitro, Amino, $C_1$—$C_2$-Alkyl, $C_1$—$C_2$-Alkoxy, $C_1$—$C_2$-Alkylthio, $C_1$—$C_2$-Alkylsulfinyl, $C_1$—$C_2$-Alkylsulfonyl, Halogen-$C_1$—$C_4$-Alkyl, Halogen-$C_1$—$C_2$-Alkoxy, Halogen-$C_1$—$C_2$-Alkylthio, Halogen-$C_1$—$C_2$-Alkylsulfonyl, $C_1$—$C_2$-Alkylcarbonyl, $C_1$—$C_2$-Alkoxycarbonyl, Aminocarbonyl, $C_1$—$C_2$-Alkoxycarbonylamino und/oder Sulfonylamino substituiertes Phenyl steht oder für den Rest

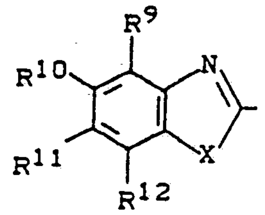

steht, wobei

$R^9$, $R^{10}$, $R^{11}$ und $R^{12}$ gleich oder verschieden sind und für Wasserstoff, Fluor, Chlor, Brom, $C_1$—$C_2$-Alkyl, $C_1$—$C_2$-Alkoxy, Halogen-$C_1$—$C_2$-Alkyl oder Halogen-$C_1$—$C_2$-Alkoxy stehen und

X für Sauerstoff, Schwefel oder eine Gruppierung —N=CH— steht,

$R^5$, $R^6$, $R^7$ und $R^8$ gleich oder verschieden sind und für Wasserstoff, Fluor, Chlor, Brom, $C_1$—$C_2$-Alkyl oder $C_1$—$C_2$-Alkoxy stehen und

Y für Sauerstoff oder Schwefel steht und

$R^1$ und $R^2$ für Wasserstoff, Fluor, Chlor, Brom oder für gegebenenfalls durch Fluor, Chlor und/oder Brom substituiertes $C_1$—$C_4$-Alkyl, $C_1$—$C_4$-Alkoxy, $C_1$—$C_4$-Alkylthio und $C_1$—$C_4$-Alkylsulfonyl, sowie für gegebenenfalls durch Fluor, Chlor, Brom, Methyl, Methoxy, Methylthio, Trifluormethyl, Trifluormethoxy und/oder Trifluormethylthio substituiertes Phenyl stehen.

Ganz besonders bevorzugt sind die neuen Verbindungen der Formel (II), in welcher

R für einen Rest

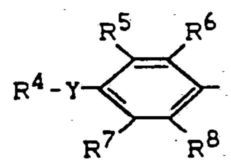

steht, wobei

$R^4$ für einen gegebenenfalls durch Fluor, Chlor, Brom, Cyano, Nitro, Trifluormethyl, Trifluormethoxy, Trifluormethylthio, Trifluorethylthio und/oder Trifluormethylsulfonyl substituiertes Phenyl steht, oder für den Rest

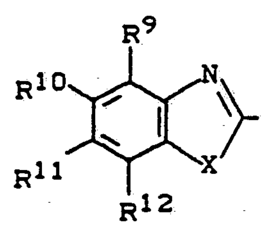

steht, wobei

$R^9$, $R^{10}$, $R^{11}$ und $R^{12}$ gleich oder verschieden sind und für Wasserstoff, Fluor, Chlor, Brom, Methyl, Methoxy, Trifluormethyl, Dichlorfluormethyl, Chlordifluormethyl, Trichlormethyl oder Trifluormethoxy stehen und

X für Sauerstoff, Schwefel oder eine Gruppierung —N=CH— steht,

$R^5$, $R^6$, $R^7$ und $R^8$ gleich oder verschieden sind und für Wasserstoff, Chlor, Methyl oder Methoxy stehen und

Y für Sauerstoff oder Schwefel steht und

$R^1$ und $R^2$ für Wasserstoff, Fluor, Chlor, Brom oder für gegebenenfalls durch Fluor, Chlor und/oder Brom substituiertes Methyl, Ethyl, Methoxy, Ethoxy, Methylthio, Ethylthio, Methylsulfonyl und Ethylsulfonyl sowie für gegebenenfalls durch Fluor, Chlor, Brom, Methyl, Methoxy, Methylthio, Trifluormethyl, Trifluormethoxy und/oder Trifluormethylthio substituiertes Phenyl stehen, wobei $R^1$ und $R^2$ nicht gleichzeitig für Wasserstoff stehen.

Im einzelnen seien folgende neue Verbindungen der Formel II genannt:

1-Methyl-5-[4-(4-phenoxy)-phenyl]-5-[3,5-dichlor-4-(4-phenoxy)-phenyl]-, -5-[3,5-dichlor-4-(4-trifluormethylsulfonyl-phenoxy)-phenyl]-, -5-[3,5-dimethyl-4-(4-trifluormethyl-sulfonyl-phenoxy)-phenyl]-, -5-[3-chlor-4-(4-trifluormethylthio-phenoxy)-phenyl]-, -5-[3-methoxy-4-(4-trifluormethylthio-phenoxy)-phenyl]-, -5-[2-Chlor-4-(4-trifluormethylthio-phenoxy)-phenyl]- und -5-[3-methyl-4-(5,6-dichlor-2-benzthiazolyloxy)-phenyl]-biuret und die entsprechenden 3-Methyl-Derivate dieser Verbindungen.

Die neuen Verbindungen der Formel (II) lassen sich gemäß dem erfindungsgemäßen Verfahren 2 herstellen (siehe weiter unten).

Die für das erfindungsgemäße Verfahren zur Herstellung der Triazintrione der Formel I außerdem als Ausgangsstoffe zu verwendenden Kohlensäuredialkylester sind durch die Formel (III) allgemein definiert.

In dieser Formel steht $R^3$ für $C_1$—$C_4$-Alkyl.

Die Verbindungen der Formel (III) sind bekannte Verbindungen der organischen Chemie.

Als Beispiele für die Verbindungen der Formel (III) seien genannt: Kohlensäuredimethylester, Kohlensäurediethylester und Kohlensäuredi-n-propylester.

Das erfindungsgemäße Verfahren zur Herstellung der Verbindungen der Formel (I) wird vorzugsweise ohne Verdünnungsmittel durchgeführt. Bevorzugt wird in Gegenwart von überschüssigem Kohlensäuredialkylester der Formel (III) gearbeitet.

Das Verfahren wird in Gegenwart von starken Basen durchgeführt. Besonders bewährt haben sich Alkalialkoholate, wie Natrium- und Kaliummethylat oder -ethylat und Kalium-tert.-butylat.

Das Verfahren wird im allgemeinen bei Temperaturen zwischen 0°C und 140°C durchgeführt. Bevorzugt wird der Bereich zwischen 20°C und 120°C. Die Umsetzungen werden im allgemeinen bei Normaldruck durchgeführt.

Zur Durchführung des Verfahrens setzt man auf 1 Mol der Verbindung der Formel (II), 1 bis 3 Mol, vorzugsweise 1 bis 2,0 Mol einer starken Base und 5 bis 30 Mol, vorzugsweise 10 bis 25 Mol Kohlensäuredialkylester der Formel (III) ein. Die Aufarbeitung erfolgt in üblicher Weise z.B. durch Zugabe von Wasser zum Reaktionsgemisch und Neutralisierung mit z.B. Salzsäure. Danach versetzt man die Mischung gegebenenfalls mit einem organischen Lösungsmittel, wie z.B. Methylenchlorid und arbeitet die organische Phase in üblicher Weise durch Waschen, Trocknen und Abdestillieren des Lösungsmittels auf.

Verwendet man beispielsweise für das erfindungsgemäße Verfahren (2a) als Ausgangsstoffe 3-Methyl-4-(4-trifluormethylthiophenoxy)-phenylisocyanat und 1-Methylharnstoff, so kann die Reaktion durch das folgende Formelschema wiedergegeben werden:

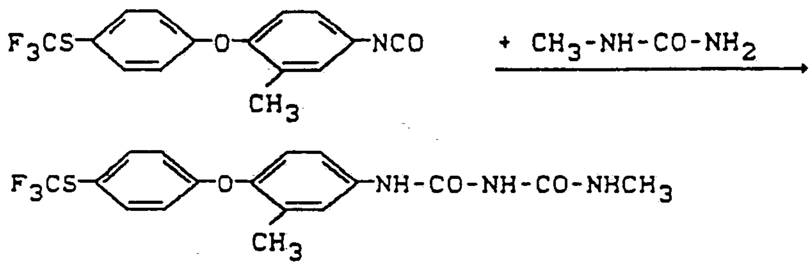

Die als Ausgangsstoffe für das erfindungsgemäße Verfahren 2a einzusetzende Verbindungen sind durch die Formel (IV) allgemein definiert. In dieser Formel steht R bevorzugt für diejenigen Reste, welche für Verbindungen der Formel (II) als bevorzugt angegeben sind. Im einzelnen seien folgende Verbindungen der Formel IV genannt:

4-(4-Phenoxy)-phenyl-, 3,5-Dichlor-4-(4-cyano-phenoxy)-phenyl-, 3,5-Dichlor-4-(4-trifluormethylsulfonyl-phenoxy)-phenyl-, 3,5-Dimethyl-4-(4-trifluormethylsulfonyl-phenoxy)-phenyl-, 3-Chlor-4-(4-trifluormethylthio-phenoxy)-phenyl-, 3-Methoxy-4-(4-trifluormethylthio-phenoxy)-phenyl-, 2-Chlor-4-(4-trifluormethylthio-phenoxy)-phenyl- und 3-Methyl-4-(5,6-dichlor-2-benzthiazolyloxy)-phenyl-isocyanat.

Die Verbindungen der Formel (IV) sind bekannt oder lassen sich nach bekannten Verfahren herstellen (vgl. z.B. EP—OS 93 976).

Die für das erfindungsgemäße Verfahren außerdem als Ausgangsstoffe zu verwendenden Verbindungen sind durch die Formel (V) allgemein definiert. In dieser Formel stehen $R^1$ und $R^2$ bevorzugt für diejenigen Reste, welche oben für Verbindungen der Formel (II) als bevorzugt angegeben sind.

Für den Fall, daß Harnstoffe der Formel (V) eingesetzt werden, in welcher $R^1$ und $R^2$ für Wasserstoff stehen, werden vorzugsweise die Biurete der Formel (II) hergestellt, in welcher $R^1$ für Wasserstoff steht. Die isomeren Biurete der Formel (II), in welcher $R^2$ für Wasserstoff steht, entstehen dabei als Nebenprodukte. Das Verhältnis dieser beiden Isomeren der Formel (II) kann durch Variation der Reactionsbedingungen geändert werden.

Die Verbindungen der Formel (V) sind bekannte Verbindungen der organischen Chemie.

Das erfindungsgemäße Verfahren (2a) zur Herstellung der Verbindungen der Formel (II) wird bevorzugt unter Verwendung von Verdünnungsmitteln durchgeführt. Als Verdünnungsmitteln kommen praktisch alle inerten organischen Lösungsmittel in Frage.

Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol, o-Dichlorbenzol, Ether wie Diethyl- und Dibutylether, Glycoldimethylether und Diglycoldimethylether, Tetrahydrofuran und Dioxan, Ketone, wie Aceton, Methylethyl-, Methylisopropyl- und Methylisobutylketon, Ester, wie Essigsäuremethylester und -ethylester und Kohlensäurediethylester, Nitrile, wie z.B. Acetonitril und Propionitril, Amide, wie z.B. Dimethylformamid, Dimethylacetamid und N-Methylpyrrolidon, sowie Dimethylsulfoxid, Tetramethylensulfon, Hexamethylphosphorsäuretriamid und Pyridin.

Die Reaktionstemperaturen können bei der Durchführung des erfindungsgemäßen Verfahrens (2a)

innerhalb eines größeren Bereiches variiert werden. Im allgemeinen arbeitet man bei Temperaturen zwischen 0°C und 140°C, vorzugsweise zwischen 0°C und 120°C. Die Umsetzungen werden im allgemeinen unter Normaldruck durchgeführt.

Bei der Durchführung des Verfahrens (2a) setzt man auf 1 Mol der Verbindung der Formel (IV) 1 bis 2 Mol, vorzugsweise 1,0 bis 1,4 Mol Harnstoff der Formel (V) ein. Die Aufarbeitung des Reaktionsproduktes geschieht nach üblichen Methoden.

Verwendet man beispielsweise für das erfindungsgemäße Verfahren (2b) als Ausgangsstoffe 3-Methyl-4-(4-trifluormethylthio-phenoxy)-anilin, Methylisocyanat, Phosgen und Ammoniak, so kann die Reaktion durch das folgende Reaktionsschema wiedergegeben werden:

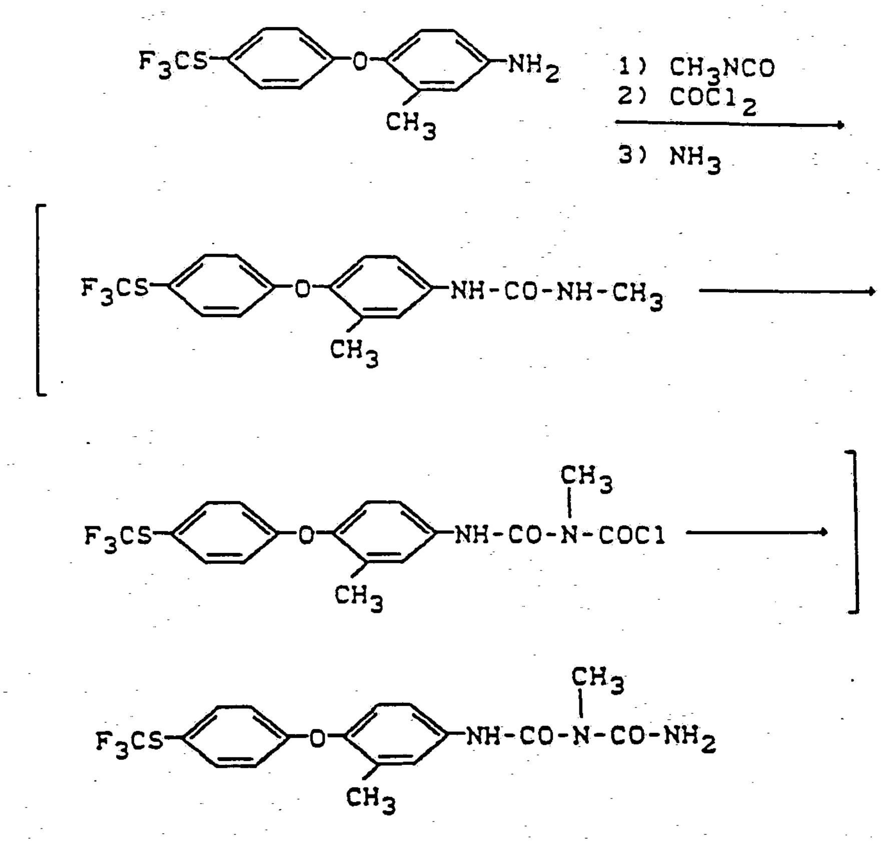


Die als Ausgangsstoffe für die 1. Stufe des erfindungsgemäßen Verfahrens (2b) einzusetzenden Amine sind durch die Formel (VI) allgemein definiert. In dieser Formel steht R bevorzugt für diejenigen Reste, welche oben für Verbindungen der Formel (II) als bevorzugt angegeben sind.

Im einzelnen seien folgende Verbindungen der Formel VI genannt:

4-Phenoxy-, 3,5-Dichlor-4-(4-Cyano-phenoxy)-, 3,5-Dichlor-4-(4-trifluormethylsulfonyl)-phenoxy)-, 3,5-Dimethyl-4-(4-trifluormethylsulfonyl-phenoxy)-, 3-Chlor-4-(4-trifluormethylthio-phenoxy)-, 3-Methoxy-4-(4-trifluormethylthio-phenoxy)-, 2-Chlor-4-(4-trifluormethylthio-phenoxy)- und 3-Methyl-4-(5,6-dichlor-2-benzthiazolyloxy)-anilin.

Die Verbindungen der Formel (VI) sind bekannt oder lassen sich nach bekannten Verfahren herstellen (vgl. z.B. DE—OS 2 413 722 und DE—OS 2 718 799).

Die Verbindung der Formel (VIa) 3-Methyl-4-(4-trifluormethylthio-phenoxy)-anilin ist neu:

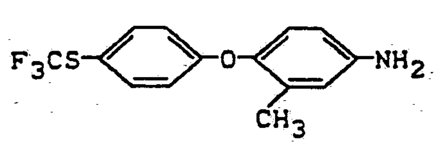

(VIa)

Diese Verbindung wird hergestellt durch Hydrierung von 3-Methyl-4-(4-trifluormethylthio-phenoxy)-nitrobenzol mit Wasserstoff, in Gegenwart von Hydrierungskatalysatoren, wie z.B. Raney-Nickel, in Gegenwart von Verdünnungsmitteln wie z.B. Toluol, bei Temperaturen zwischen 20°C und 150°C und bei einem Druck zwischen 5 und 150 bar.

3-Methyl-4-(4-trifluormethylthio-phenoxy)-nitrobenzol ist ebenfalls neu und kann z.B. hergestellt werden durch Umsetzung von 4-Trifluormethylthiophenol mit 2-Methyl-4-nitro-chlorbenzol in Gegenwart

von Säureakzeptoren, wie z.B. Natriumhydroxid und in Gegenwart von Verdünnungsmitteln, wie z.B. Dimethylsulfoxid, bei Temperaturen zwischen 0°C und 200°C.

4-Trifluormethylthiophenol und 2-Methyl-4-nitro-chlorbenzol sind allgemein bekannte Verbindungen der organischen Chemie.

Die weiterhin als Ausgangsstoffe für die 1. Stufe des erfindungsgemäßen Verfahrens (2b) einzusetzenden Verbindungen sind durch die Formel (VII) allgemein definiert.

In dieser Formel steht $R^1$ bevorzugt für diejenigen Reste, welche oben für Verbindungen der Formel (II) als bevorzugt angegeben sind. Im einzelnen seien folgende Verbindungen der Formel VII genannt:

Methylisocyanat, Ethylisocyanat, n-Propylisocyanat, Phenylisocyanat, 4-Chlorphenylisocyanat.

Die Verbindungen der Formel (VII) sind allgemein bekannte Verbindungen der organischen Chemie.

Die als Ausgangsstoffe für die 2. Stufe des erfindungsgemäßen Verfahrens (2b) einzusetzenden Verbindungen sind durch die Formel (VIII) allgemein definiert. In dieser Formel stehen R und $R^1$ bevorzugt für diejenigen Reste, welche oben für Verbindungen der Formel (II) als bevorzugt angegeben sind. Im einzelnen seien folgende Verbindungen der Formel VIII genannt:

1-Methyl-3-[4-(4-phenoxy)-phenyl]-, -3-[3,5-dichlor-4-(4-cyano-phenoxy)-phenyl], -3-[3,5-dichlor-4-(4-trifluormethylsulfonyl-phenoxy)-phenyl]-, -3-[3,5-dimethyl-4-(4-trifluormethylsulfonyl-phenoxy)-phenyl]-, -3-[3-Chlor-4-(4-trifluormethylthio-phenoxy)-phenyl]-, -3-[3-methoxy-4-(4-trifluormethylthio-phenoxy)-phenyl]-, -3-[2-chlor-4-(4-trifluormethylthio-phenoxy)-phenyl]- und -3-[3-methyl-4-(5,6-dichlor-2-benzthiazolyloxy)-phenyl]-harnstoff.

Die Verbindungen der Formel (VIII) sind bekannt oder lassen sich nach bekannten Verfahren herstellen (vgl. z.B. DE—OS 2 413 722 und DE—OS 2 718 799).

Die als Ausgangsstoffe für die 3. Stufe des erfindungsgemäßen Verfahrens (2b) einzusetzenden Verbindungen sind durch die Formel (IX) allgemein definiert. In dieser Formel stehen R und $R^1$ bevorzugt für diejenigen Reste, welche oben für Verbindungen der Formel (II) als bevorzugt angegeben sind.

Die Verbindungen der Formel (IX) sind zum Teil bekannt (vgl. z.B. "Methoden der organischen Chemie", Band E IV, S. 1026 Houben-Weyl-Müller, Verlag Stuttgart).

N-Chlorcarbonylharnstoffe der Formel (IX) in welcher die Reste R und $R^1$ die gemäß 1 (oben) angegebene Bedeutung besitzen sind neu mit Ausnahme von N-4(4-chlorphenoxy)-phenyl-N'-methyl-N'-chlorcarbonyl-harnstoff.

Bevorzugt sind die neuen Verbindungen der Formel (IX), in welcher

R für einen Rest

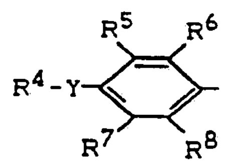

steht, wobei

$R^4$ für gegebenenfalls durch Fluor, Chlor, Brom, Cyano, Nitro, Amino, $C_1$—$C_2$-Alkyl, $C_1$—$C_2$-Alkoxy, $C_1$—$C_2$-Alkylthio, $C_1$—$C_2$-Alkylsulfinyl, $C_1$—$C_2$-Alkylsulfonyl, Halogen-$C_1$—$C_2$-Alkyl, Halogen-$C_1$—$C_2$-Alkoxy, Halogen-$C_1$—$C_2$-Alkylthio, Halogen-$C_1$—$C_2$-Alkylsulfonyl, Hydroxycarbonyl, $C_1$—$C_2$-Alkylcarbonyl, $C_1$—$C_2$-Alkoxycarbonyl, Aminocarbonyl, $C_1$—$C_2$-Alkoxycarbonylamino und/oder Sulfonylamino substituiertes Phenyl steht oder für den Rest

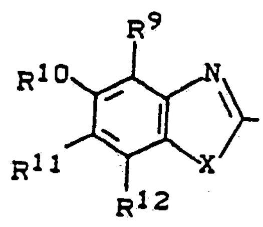

steht, wobei

$R^9$, $R^{10}$, $R^{11}$ und $R^{12}$ gleich oder verschieden sind und für Wasserstoff, Fluor, Chlor, Brom, $C_1$—$C_2$-Alkyl, $C_1$—$C_2$-Alkoxy, Halogen-$C_1$—$C_2$-Alkyl oder Halogen-$C_1$—$C_2$-Alkoxy stehen und

X für Sauerstoff, Schwefel oder eine Gruppierung —N=CH— steht,

$R^5$, $R^6$, $R^7$ und $R^8$ gleich oder verschieden sind und für Wasserstoff, Fluor, Chlor, Brom, $C_1$—$C_2$-Alkyl oder $C_1$—$C_2$-Alkoxy stehen und

Y für Sauerstoff oder Schwefel steht und

$R^1$ für Wasserstoff, Fluor, Chlor, Brom oder für gegebenenfalls durch Fluor, Chlor und/oder Brom substituiertes $C_1$—$C_4$-Alkyl, $C_1$—$C_4$-Alkoxy, $C_1$—$C_4$-Alkylthio und $C_1$—$C_4$-Alkylsulfonyl, sowie für gegebenenfalls durch Fluor, Chlor, Methyl, Methoxy, Methylthio, Trifluormethyl, Trifluormethoxy und/oder Trifluormethylthio substituiertes Phenyl steht.

Ganz besonders bevorzugt sind die neuen Verbindungen der Formel (IX), in welcher

R für einen Rest

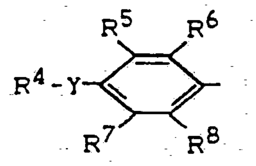

steht, wobei

$R^4$ für einen gegebenenfalls durch Fluor, Chlor, Brom, Cyano, Nitro, Trifluormethyl, Trifluormethoxy, Trifluormethylthio, Trifluorethylthio und/oder Trifluormethylsulfonyl substituiertes Phenyl steht, oder für den Rest

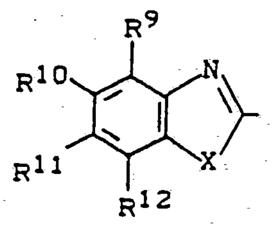

$R^9$, $R^{10}$, $R^{11}$ und $R^{12}$ gleich oder verschieden sind und für Wasserstoff, Fluor, Chlor, Brom, Methyl, Methoxy, Trifluormethyl, Dichlorfluormethyl, Chlordifluormethyl, Trichlormethyl oder Trifluormethoxy stehen und

X für Sauerstoff, Schwefel oder eine Gruppierung —N=C— steht,

$R^5$, $R^6$, $R^7$ und $R^8$ gleich oder verschieden sind und für Wasserstoff, Chlor, Methyl oder Methoxy stehen und

Y für Sauerstoff oder Schwefel steht und

$R^1$ für Wasserstoff, Fluor, Chlor, Brom oder für gegebenenfalls durch Fluor, Chlor und/oder Brom substituiertes Methyl, Ethyl, Methoxy, Ethoxy, Methylthio, Ethylthio, Methylsulfonyl und Ethylsulfonyl sowie für gegebenenfalls durch Fluor, Chlor, Brom, Methyl, Methoxy, Methylthio, Trifluormethyl, Trifluormethoxy und/oder Trifluormethylthio substituiertes Phenyl steht.

Im einzelnen sein folgende Verbindungen der Formel IX genannt:

1-Chlorcarbonyl-1-methyl-3-[4-(4-phenoxy)-phenyl]-3-[3,5-dichlor-4-(4-cyano-phenoxy)-phenyl], -3-[3,5-dichlor-4-(4-trifluormethylsulfonyl-phenoxy)-phenyl]-, -3-[3,5-dimethyl-4-(4-trifluormethylsulfonyl-phenoxy)-phenyl]-, -3-[3-chlor-4-(4-trifluormethylthio-phenoxy)-phenyl]-, -3-[3-methoxy-4-(4-trifluormethylthio-phenoxy)-phenyl]-, -3-[2-chlor-4-(4-trifluormethylthio-phenoxy)-phenyl]- und -3-[3-methyl-4-(5,6-dichlor-2-benzthiazolyloxy)-phenyl]-harnstoff.

Die N-Chlorcarbonylharnstoffe der Formel (IX) lassen sich entsprechend Stufe 2 des erfindungsgemäßen Verfahrens (3b) herstellen und werden anschließend gemäß Stufe 3 des erfindungsgemäßen Verfahrens (3b) zu den Verbindungen der Formel (II), in welcher $R^2$ für Wasserstoff steht, umgesetzt.

Das erfindungsgemäße Verfahren (3b) wird in allen drei Stufen bevorzugt unter Verwendung von Verdünnungsmitteln durchgeführt. Als Verdünnungsmittel kommen die organischen Lösungsmittel in Frage, die bereits für das erfindungsgemäße Verfahren (3b) genannt worden sind.

Die Reaktionstemperaturen können bei der Durchführung des erfindungsgemäßen Verfahrens (3b) in allen drei Stufen innerhalb eines größeren Bereiches variiert werden.

Im allgemeinen arbeitet man in allen Stufen bei Temperaturen zwischen 0°C und 100°C, vorzugsweise zwischen 15°C und 80°C. Die Umsetzungen werden im allgemeinen unter Normaldruck durchgeführt.

Bei der Durchführung des Verfahrens (3b) setzt man in der 1. Stufe auf 1 Mol Amin der Formel (VI), 1 bis 2 Mol, vorzugsweise 1 bis 1,5 Mol der Verbindung der Formel (VII), anschließend in der 2. Stufe, gegebenenfalls nach Isolierung der Verbindung der Formel (VIII), gibt man 1,0 bis 3,0 Mol, vorzugsweise 1,5 bis 2,5 Mol Phosgen zu und danach in der 3. Stufe gegebenenfalls nach Isolierung der N-Chlorcarbonylharnstoffe der Formel (IX), wird bis zum vollständigen Umsatz der Verbindungen der Formel (IX) Ammoniak eingeleitet. Die Aufarbeitung geschieht nach üblichen Methoden.

Die einzelnen Stufen des erfindungsgemäßen Verfahrens (3b) lassen sich auch getrennt durchführen. Dabei werden nach jeder stufe die entstandenen Verbindungen isoliert und gereinigt. Sie können dann weiter umgesetzt werden oder anderweitig eingesetzt werden.

Die Verbindungen der Formel (I), die nach dem erfindungsgemäßen Verfahren (4) leicht zugänglich sind, weisen eine ausgezeichnete das Wachstum von Tieren fördernde und eine hervorragende coccidiostatische Wirkung auf (vgl. z.B. DE—OS 2 413 722 und DE—OS 2 718 799).

## Herstellungsbeispiele

### Beispiel 1

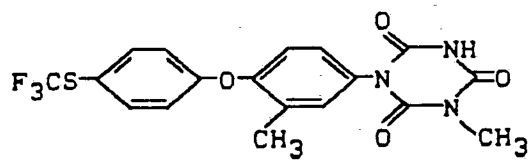

399 g (1 Mol) 1-Methyl-5-[3-methyl-4-(4-trifluormethylthio-phenoxy)-phenyl]-biuret, 108 g (2 Mol) Natriummethylat und 2400 ml Diethylcarbonat werden 3 Stunden unter Rückfluß erhitzt. Der entstehende Alkohol wird gleichzeitig abdestilliert. Man kühlt auf 25°C ab, gibt 4800 g Wasser zu, neutralisiert mit Salzsäure und trennt die organische Phase ab. Die wäßrige Phase wird einmal mit Diethylcarbonat extrahiert. Die vereinigten organischen Phasen werden im Wasserstrahlvakuum eingeengt.

Man erhält 421 g (94% der Theorie) 1-[3-Methyl-4-(4-trifluormethylthio-phenoxy)-phenyl]-3-methyl-1,3,5-triazin-2,4,6-trion mit einer 95%igen Reinheit und vom Schmelzpunkt 191°C (Ethanol).

### Beispiel 2

2 g (0,005 Mol) 99,3%iges 3-Methyl-5-[3-methyl-4-(4-trifluormethylthio-phenoxy)-phenyl]-biuret, 0,7 g (0,01 Mol) Natriummethylat und 10 g Diethylcarbonat werden 3 Stunden bei 20°C gerührt. Danach wird Wasser zugesetzt, mit Salzsäure neutralisiert und mit Methylenchlorid extrahiert. Die organische Phase wird abgetrennt, getrocknet und eingeengt.

Nach dem Andestillieren erhält man 2,1 g (100% der Theorie) 1-[3-Methyl-4-(4-trifluormethylthio-phenoxy)-phenyl]-3-methyl-1,3,5-triazin-2,4,6-trion mit einer 97,3%igen Reinheit und vom Schmelzpunkt 190°C.

Ausgangsprodukte der Formel (II)

### Beispiel 3

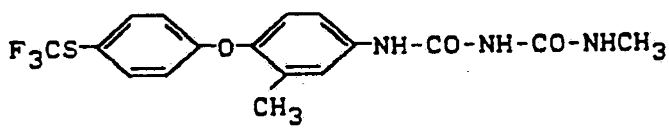

(Verfahren (3))

325,3 g (1 Mol) 3-Methyl-4-(4-trifluormethylthio-phenoxy)-phenylisocyanat, 74,1 g (1 Mol) Methylharnstoff und 95 ml Methylenchlorid werden 3 Stunden unter Rückfluß erhitzt. Anschließend werden 230 ml Methylenchlorid zugegeben und auf 0°C abgekühlt. Das Produkt wird abfiltriert und getrocknet.

Man erhält auf diese Weise 298 g (75% der Theorie) 1-Methyl-5-[3-methyl-4-(4-trifluormethylthio-phenoxy)-phenyl]-biuret vom Schmelzpunkt 153°C (Benzin).

### Beispiel 4

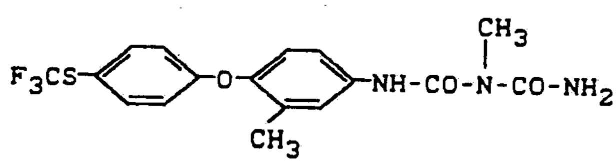

(Verfahren (4) "Eintopf-Verfahren")

Zu 4,2 g (0,014 Mol) 3-Methyl-4-(4-trifluormethylthio-phenoxy)-anilin in 40 ml Toluol werden 0,84 g (0,015 Mol) Methylisocyanat bei 20°C zugetropft und ca. 12 Stunden nachgerührt. Anschließend werden 12,5 g 20%ige Phosgen-Lösung in Toluol (0,025 Mol) zugetropft, 8 Stunden bei 20°C nachgerührt und anschließend 3 Stunden auf 60°C erhitzt. Nach Kontrolle per Dünnschicht-Chromatogramm auf vollständigen Umsatz leitet man durch diese Lösung bei 20°C so lange Ammoniak ein, bis sich dünnschichtchromatographisch kein N-Chlorcarbonylharnstoff mehr nachweisen läßt. Anschließend wird abgesaugt, der Rückstand wird gut mit Wasser verrührt, filtriert und getrocknet.

Man erhält 5,2 g (93% der Theorie) 3-Methyl-5-[3-methyl-4-(4-trifluormethylthio-phenoxy)-phenyl]-biuret mit einer 98,5%igen Reinheit vom Schmelzpunkt 182—183°C.

Beispiel 5

Durch eine Lösung von 4,2 g (0,01 Mol) 1-Chlorcarbonyl-1-methyl-3-[3-methyl-4-(4-trifluormethylthio-phenoxy)-phenyl]-harnstoff (vgl. Beispiel 9) in 20 ml Toluol wird unterhalb von 20°C so lange Ammoniak eingeleitet, bis kein Ausgangsprodukt mehr vorhanden ist.

Anschließend wird abgesaugt und der Rückstand mit 40 ml Wasser verrührt, filtriert und getrocknet.

Man erhält so 3,8 g (95% der Theorie) 3-Methyl-5-[3-methyl-4-(4-trifluormethylthio-phenoxy)-phenyl]-biuret mit einer 99%igen Reinheit vom Schmelzpunkt 182°C.

Ausgangsverbindung der Formel (IV)

Beispiel 6

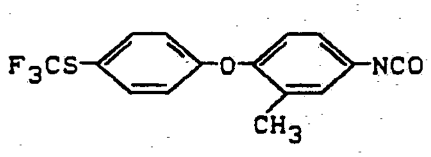

480 g Chlorbenzol und 180 g Phosgen werden bei 0°C vorgelegt und innerhalb von 30 Minuten mit einer Lösung von 300 g (1 Mol) 3-Methyl-4-(4-trifluormethylthio-phenoxy)-anilin in 550 g Chlorbenzol versetzt, wobei die Temperatur auf 25°C ansteigt. Anschließend wird innerhalb von 1 Stunde bis zum Siedepunkt erhitzt, wobei ab 80°C weiterhin Phosgen eingeleitet wird, bis kein Chlorwasserstoff mehr nachweisbar ist (ca. 1 Stunde).

Das überschüssige Phosgen wird mit Stickstoff ausgeblasen, das Lösungsmittel abgezogen und das Rohprodukt destilliert.

Man erhält 316 g (96% der Theorie) 3-Methyl-4-(4-trifluormethylthio-phenoxy)-phenylisocyanat mit einer 99%igen Reinheit und vom Siedepunkt Kp: 162°C/2 mbar.

Ausgangsverbindung der Formel (VI)

Beispiel 7

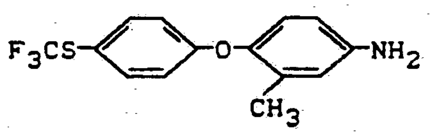

329,3 g (1 Mol) 3-Methyl-4-(4-trifluormethylthio-phenoxy)-nitrobenzol wird in 1100 g Toluol gelöst und nach Zugabe von 20 g Raney-Nickel bei 90°C und einem Wasserstoffdruck von 90 bar hydriert. Nach Ende der Wasserstoffaufnahme wird der Katalysator abfiltriert, das Lösungsmittel abdestilliert und das Product im Ölpumpenvakuum rektifiziert.

Man erhält 270,7 g (90% der Theorie) 3-Methyl-4-(4-trifluormethylthio-phenoxy)-anilin mit einer Reinheit von 99,5% und einem Schmelzpunkt von 44—45°C.

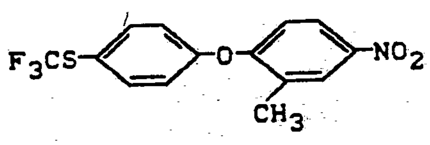

198,1 g (1,02 Mol) 4-Trifluormethylthiophenol werden in 330 g Dimethylsulfoxid vorgelegt. Bei 20°C werden 44,0 g (1,1 Mol) Natriumhydroxid-Schuppen zugegeben und unter Erhitzen auf 90°C aufgelöst. In 30 Minuten werden 171,6 g (1 Mol) 2-Methyl-4-nitro-chlorbenzol gelöst in 330 g Dimethylsulfoxid zugegeben. Anschließend wird die Temperatur von 90°C auf 140°C erhöht und 3 Stunden nachgerührt. Innerhalb von 2 Stunden wird bei 140°C das Lösungsmittel abdestilliert, wobei der Druck allmählich auf 10 bis 20 mbar abgesenkt wird. Dann wird der Rückstand auf 20°C abgekühlt und mit 1 l Wasser versetzt. Die entstandene Suspension wird filtriert, der Niederschlag wird mit verdünnter Natronlauge und Wasser gewaschen, getrocknet und aus Petrolether umkristallisiert.

Man erhält 281,3 g (85% der Theorie) 3-Methyl-4-(4-trifluormethylthio-phenoxy)-nitrobenzol mit einer Reinheit von 99,5% und einem Schmelzpunkt von 61—62°C.

Ausgangsverbindung der Formel (VIII)

Beispiel 8

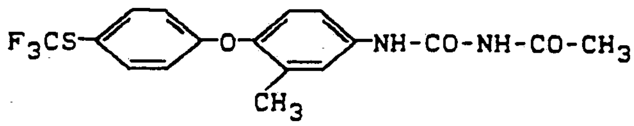

74,8 g (2,5 Mol) 3-Methyl-4-(4-trifluormethylthio-phenoxy)-anilin werden in 2170 g wasserfreiem Toluol gelöst und bei 40°C innerhalb von 2 Stunden mit 157 g (2,75 Mol) Methylisocyanat versetzt und anschließend noch 3 Stunden nachgerührt. Das überschüssige Methylisocyanat wird mit 600 g Toluol im Vakuum abdestilliert. Der Rückstand wird dann noch 1 Stunden bei 10°C gerührt. Das Produkt wird abfiltriert, mit Toluol von 10°C gewaschen und getrocknet.

Man erhält 881,9 g (98% der Theorie) 1-Methyl-3-[3-methyl-4-(4-trifluormethylthio-phenoxy)-phenyl]-harnstoff mit einer Reinheit von 99% und einem Schmelzpunkt von 129—130°C.

Ausgangsverbindung der Formel (IX)

Beispiel 9

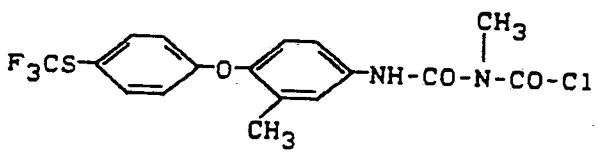

7,1 g (0,02 Mol) 1-Methyl-3-[3-methyl-4-(4-trifluormethylthio-phenoxy)-phenyl]-harnstoff in 25 ml Toluol werden bei 20°C tropfenweise mit 17,8 g einer 20%igen Lösung (0,036 Mol) von Phosgen in Toluol versetzt, 15 bis 16 Stunden bei 20°C nachgerührt und anschließend noch 4 Stunden auf 50°C bis 60°C erhitzt.

Nach Einengen und Andestillieren erhält man 8,4 g (100% der Theorie) 1-Chlorcarbonyl-1-methyl-3-[3-methyl-4-(4-trifluormethylthio-phenoxy)-phenyl]-harnstoff mit einer Reinheit von 94% und einem Schmelzpunkt von 79°C.

**Patentansprüche**

1. Biurete der Formel (II)

$$R—NH—CO—NR^1—CO—NHR^2 \qquad (II)$$

in welcher

R für einen Rest

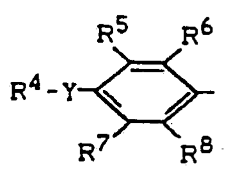

steht, wobei

$R^4$ für gegebenenfalls durch Halogen, Cyano, Nitro, Amino, $C_1$—$C_4$-Alkyl, $C_1$—$C_4$-Alkoxy, $C_1$—$C_4$-Alkylthio, $C_1$—$C_4$-Alkylsulfinyl, $C_1$—$C_4$-Alkylsulfonyl, Halogen-$C_1$—$C_4$-Alkyl, Halogen-$C_1$—$C_4$-Alkoxy, Halogen-$C_1$—$C_4$-Alkylthio, Halogen-$C_1$—$C_4$-Alkylsulfonyl, Hydroxycarbonyl, $C_1$—$C_4$-Alkylcarbonyl, $C_1$—$C_4$-Alkoxycarbonyl, Aminocarbonyl, $C_1$—$C_4$-Alkoxycarbonylamino und/oder Sulfonylamino substituiertes Phenyl steht oder für den Rest

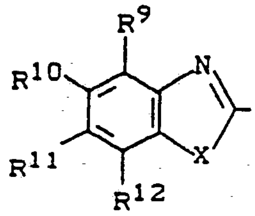

steht, wobei

$R^9$, $R^{10}$, $R^{11}$ und $R^{12}$ gleich oder verschieden sind und für Wasserstoff, Halogen $C_1$—$C_4$-Alkyl, $C_1$—$C_4$-Alkyl, $C_1$—$C_4$-Alkoxy, Halogen-$C_1$—$C_4$-Alkyl/oder Halogen-$C_1$—$C_4$-Alkoxy stehen und

X für Sauerstoff, Schwefel oder eine Gruppierung —N=CH— steht,

$R^5$, $R^6$, $R^7$ und $R^8$ gleich oder verschieden sind und für Wasserstoff, Halogen, $C_1$—$C_4$-Alkyl oder $C_1$—$C_4$-Alkoxy stehen und

Y für Sauerstoff, Schwefel oder die Gruppierungen —CO—, —SO—, $SO_2$—, —$CH_2$—, —$CH_2O$— oder —$OCH_2$— steht und

$R^1$ und $R^2$ für Wasserstoff, Halogen, für gegebenenfalls durch Halogen substituiertes $C_1$—$C_6$-Alkyl, $C_1$—$C_6$-Alkoxy, $C_1$—$C_6$-Alkylthio und $C_1$—$C_6$-Alkylsulfonyl, sowie für gegebenenfalls durch Halogen, $C_1$—$C_2$-Alkyl, $C_1$—$C_2$-Alkoxy, $C_1$—$C_2$-Alkylthio, Halogen-$C_1$—$C_2$-Alkyl, Halogen-$C_1$—$C_2$-Alkoxy und/oder Halogen-$C_1$—$C_2$-Alkylthio substituiertes Phenyl stehen.

2. Verfahren zur Herstellung der Biurete der Formel (II)

$$R—NH—CO—NR^1—CO—NHR^2 \qquad (II)$$

in welcher

R für einen Rest

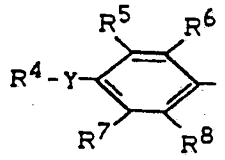

steht, wobei

$R^4$ für gegebenenfalls durch Halogen, Cyano, Nitro, Amino, $C_1$—$C_4$-Alkyl, $C_1$—$C_4$-Alkoxy, $C_1$—$C_4$-Alkylthio, $C_1$—$C_4$-Alkylsulfinyl, $C_1$—$C_4$-Alkylsulfonyl, Halogen-$C_1$—$C_4$-Alkyl, Halogen-$C_1$—$C_4$-Alkoxy, Halogen-$C_1$—$C_4$-Alkylthio, Halogen-$C_1$—$C_4$-Alkylsulfonyl, Hydroxycarbonyl, $C_1$—$C_4$-Alkylcarbonyl, $C_1$—$C_4$-Alkoxycarbonyl, Aminocarbonyl, $C_1$—$C_4$-Alkoxycarbonylamino und/oder Sulfonylamino substituiertes Phenyl steht oder für den Rest

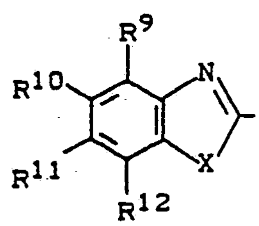

steht, wobei

$R^9$, $R^{10}$, $R^{11}$ und $R^{12}$ gleich oder verschieden sind und für Wasserstoff, Halogen $C_1$—$C_4$-Alkyl, $C_1$—$C_4$-Alkyl, $C_1$—$C_4$-Alkoxy, Halogen-$C_1$—$C_4$-Alkyl/oder Halogen-$C_1$—$C_4$-Alkoxy stehen und

X für Sauerstoff, Schwefel oder eine Gruppierung —N=CH— steht,

$R^5$, $R^6$, $R^7$ und $R^8$ gleich oder verschieden sind und für Wasserstoff, Halogen, $C_1$—$C_4$-Alkyl oder $C_1$—$C_4$-Alkoxy stehen und

Y für Sauerstoff, Schwefel oder die Gruppierungen —CO—, —SO—, $SO_2$—, —$CH_2$—, —$CH_2O$— oder —$OCH_2$— steht und

$R^1$ und $R^2$ für Wasserstoff, Halogen, für gegebenenfalls durch Halogen substituiertes $C_1$—$C_6$-Alkyl, $C_1$—$C_6$-Alkoxy, $C_1$—$C_6$-Alkylthio und $C_1$—$C_6$-Alkylsulfonyl, sowie für gegebenenfalls durch Halogen, $C_1$—$C_2$-Alkyl, $C_1$—$C_2$-Alkoxy, $C_1$—$C_2$-Alkylthio, Halogen-$C_1$—$C_2$-Alkyl, Halogen-$C_1$—$C_2$-Alkoxy und/oder Halogen-$C_1$—$C_2$-Alkylthio substituiertes Phenyl stehen das dadurch gekennzeichnet ist, daß man

a) Isocyanate der Formel (IV)

$$R—NCO \qquad (IV)$$

in welcher

R die oben angegebene Bedeutung hat, mit Harnstoffen der Formel (V)

$$R^1NH—CO—NHR^2 \quad (V)$$

in welcher

$R^1$ und $R^2$ die angegebenen Bedeutungen haben, gegebenenfalls in Gegenwart von Verdünnungsmitteln umsetzt, oder

b) für den Fall, daß Verbindungen der Formel II hergestellt werden in denen

$R^2$ für Wasserstoff steht, dadurch gekennzeichnet, daß man in der 1. Stufe Amine der Formel (VI),

$$R—NH_2 \quad (VI)$$

in welcher R die oben angegebenen Bedeutungen haben, mit Verbindungen der Formel (VII)

$$R^1NCO \quad (VII)$$

in welcher $R^1$ die oben angegebenen Bedeutungen hat, in Gegenwart von inerten Verdünnungsmitteln bei Temperaturen zwischen 0°C und 100°C zu den Verbindungen der Formel (VIII)

$$R—NH—CO—NR^1H \quad (VIII)$$

in welcher R und $R^1$ die oben angegebenenen Bedeutungen haben, umsetzt und anschließend gegebenenfalls nach ihrer Isolierung, in einer 2. Stufe mit Phosgen, gegebenenfalls in Gegenwart von inerten Verdünnungsmitteln bei Temperaturen zwischen 0°C und 100°C zu den N-Chlorcarbonylharntoffen der Formel (IX)

$$R—NH—CO—NR^1—COCl \quad (IX)$$

in welcher R und $R^1$ die oben angegebenenen Bedeutungen haben, umsetzt und anschließend gegebenenfalls nach ihrer Isolierung in einer 3. Stufe, mit Ammoniak, gegebenenfalls in Gegenwart von inerten Verdünnungsmitteln bei Temperaturen zwischen 0°C und 100°C umsetzt.

3. Verwendung der Biurete der Formel II gemäß Anspruch 1 zur Herstellung von 1,3,5-Triazintrionen der Formel I

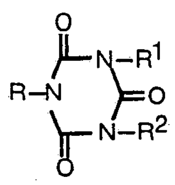

I

in welcher R, $R^1$, $R^2$ die in Anspruch 1 angegebene Bedeutung haben, dadurch gekennzeichnet, daß man die Biurete der Formel II mit Kohlensäuredialkylestern der Formel III

$$(R^3O)_2CO \quad III$$

in welcher $R^3$ für Alkyl steht, in Gegenwart von starken Basen, gegebenenfalls in Gegenwart von Verdünnungsmitteln umsetzt.

4. N-Chlorcarbonylharnstoffe der Formel (IX)

$$R—NH—CO—NR^1—COCl \quad (IX)$$

in welcher die Reste R und $R^1$ die in Anspruch 1 angegebene Bedeutung besitzen, ausgenommen N-4(4-chlorphenoxy)-phenyl-N'-methyl-N'-chlorcarbonylharnstoff.

5. Verfahren zur Herstellung der N-Chlorcarbonylharnstoffe der Formel IX gemäß Anspruch 5, dadurch gekennzeichnet, daß man Verbindungen der Formel VIII

$$R—NH—CO—NR^1H \quad (VIII)$$

in welcher R und $R^1$ die in Anspruch 5 angegebene Bedeutung haben, mit Phosgen umsetzt.

**Revendications**

1. Biurets de formule (II)

$$R—NH—CO—NR^1—CO—NHR^2 \quad (II)$$

dans laquelle
R est un reste

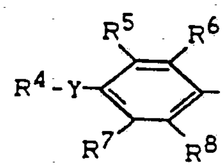

dans lequel
$R^4$ est un groupe phényle éventuellement substitué par un halogène, un radical cyano, nitro, amino, alkyle en $C_1$ à $C_4$, alkoxy en $C_1$ à $C_4$, alkythio en $C_1$ à $C_4$, alkylsulfinyle en $C_1$ à $C_4$, alkylsulfonyle en $C_1$ à $C_4$, halogénalkyle en $C_1$ à $C_4$, halogénalkoxy en $C_1$ à $C_4$, halogénalkylthio en $C_1$ à $C_4$, halogénalkylsulfonyle en $C_1$ à $C_4$, hydroxycarbonyle, (alkyle en $C_1$ à $C_4$)-carbonyle, (alkoxy en $C_1$ à $C_4$)-carbonyle, aminocarbonyle, (alkoxy en $C_1$ à $C_4$)-carbonylamino et/ou sulfonylamino, ou bien le reste

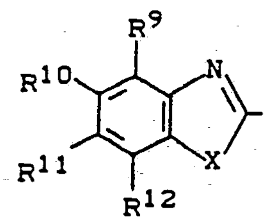

dans lequel
$R^9$, $R^{10}$, $R^{11}$ et $R^{12}$ sont identiques ou différents et représentent l'hydrogène, un halogène, un groupe alkyle en $C_1$ à $C_4$, alkyle en $C_1$ à $C_4$, alkoxy en $C_1$ à $C_4$, halogénalkyle en $C_1$ à $C_4$ ou halogénalkoxy en $C_1$ à $C_4$ et
X est l'oxygène, le soufre ou un groupement —N=CH—,
$R^5$, $R^6$, $R^7$ et $R^8$ sont identiques ou différents et représentent l'hydrogène, un halogène, un groupe alkyle en $C_1$ à $C_4$ ou alkoxy en $C_1$ à $C_4$ et
Y est l'oxygène, le soufre ou le groupement —CO—, —SO—, $SO_2$—, —$CH_2$—, —$CH_2O$— ou —$OCH_2$— et
$R^1$ et $R^2$ représentent l'hydrogène, un halogène, un groupe alkyle en $C_1$ à $C_6$, alkoxy en $C_1$ à $C_6$, alkylthio en $C_1$ à $C_6$, alkylsulfonyle en $C_1$ à $C_6$ éventuellement substitué par un halogène, ainsi qu'un groupe phényle éventuellement substitué par un halogène, un radical alkyle en $C_1$ ou $C_2$, alkoxy ou $C_1$ ou $C_2$, alkylthio ou $C_1$ ou $C_2$, halogénalkyle ou $C_1$ ou $C_2$, halogénalkoxy en $C_1$ ou $C_2$ et/ou halogénalkylthio en $C_1$ ou $C_2$.

2. Procédé de préparation des biurets de formule (II)

$$R—NH—CO—NR^1—CO—NHR^2 \quad (II)$$

dans laquelle
R est un reste

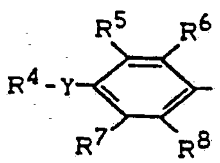

dans lequel
$R^4$ est un groupe phényle éventuellement substitué par un halogène, un radical cyano, nitro, amino, alkyle en $C_1$ à $C_4$, alkoxy en $C_1$ à $C_4$, alkylthio en $C_1$ à $C_4$, alkylsulfinyle en $C_1$ à $C_4$, alkylsulfonyl en $C_1$ à $C_4$, halogénalkyle en $C_1$ à $C_4$, halogénalkoxy en $C_1$ à $C_4$, halogénalkylthio en $C_1$ à $C_4$, halogénalkylsulfonyle en $C_1$ à $C_4$, hydroxycarbonyle, (alkyle en $C_1$ à $C_4$)-carbonyle, (alkoxy en $C_1$ à $C_4$)-carbonyle, aminocarbonyle, (alkoxy en $C_1$ à $C_4$)-carbonylamino et/ou sulfonylamino, ou bien le reste

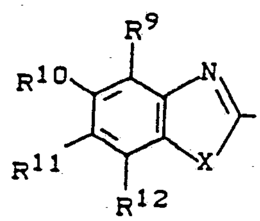

dans lequel

$R^9$, $R^{10}$, $R^{11}$ et $R^{12}$ sont identiques ou différents et représentent l'hydrogène, un halogène, un groupe alkyle en $C_1$ à $C_4$, alkyle en $C_1$ à $C_4$, alkoxy en $C_1$ à $C_4$, halogénalkyle en $C_1$ à $C_4$ ou halogénalkoxy en $C_1$ à $C_4$ et

X est l'oxygène, le soufre ou un groupement —N=CH—,

$R^5$, $R^6$, $R^7$ et $R^8$ sont identiques ou différents et représentent l'hydrogène, un halogène, un groupe alkyle en $C_1$ à $C_4$ ou alkoxy en $C_1$ à $C_4$ et

Y est l'oxygène, le soufre ou le groupement —CO—, —SO—, $SO_2$—, —$CH_2$—, —$CH_2O$— ou —$OCH_2$— et

$R^1$ et $R^2$ représentent l'hydrogène, un halogène, un groupe alkyle en $C_1$ à $C_6$, alkoxy en $C_1$ à $C_6$, alkythio en $C_1$ à $C_6$, alkylsulfonyle en $C_1$ à $C_6$ éventuellement substitué par un halogène, ainsi qu'un groupe phényle éventuellement substitué par un halogène, un radical alkyle en $C_1$ ou $C_2$, alkoxy en $C_1$ ou $C_2$, alkylthio en $C_1$ ou $C_2$, halogénalkyle en $C_1$ ou $C_2$, halogénalkoxy en $C_1$ ou $C_2$ et/ou halogénalkylthio en $C_1$ ou $C_2$, qui est caractérisé en ce que

a) on fait réagir des isocyanates de formule (IV)

$$R—NCO \qquad (IV)$$

dans laquelle

R a la définition indiquée ci-dessus avec des urées de formule (V)

$$R^1NH—CO—NHR^2 \qquad (V)$$

dans laquelle

$R^1$ et $R^2$ ont les définitions indiquées, le cas échéant en présence de diluants, ou bien

b) au cas où l'on prépare des composés de formule II dans lesquels

$R^2$ représente l'hydrogène, caractérisé en ce qu'on fait réagir dans la première étape des amines de formule (VI)

$$R—NH_2 \qquad (VI)$$

dans laquelle R a les définitions indiquées, avec des composés de formule (VII)

$$R^1NCO \qquad (VII)$$

dans laquelle $R^1$ a les définitions indiquées, en présence de diluants inertes, à des températures comprises entre 0°C et 100°C pour former les composés de formule (VIII)

$$R—NH—CO—NR^1H \qquad (VIII)$$

dans laquelle R et $R^1$ ont les définitions indiquées, puis on fait réagir ces composés, éventuellement après les avoir isolées, dans une deuxième étape avec du phosgène, éventuellement en présence de diluants inertes à des températures comprise entre 0°C et 100°C pour former les N-chlorocarbonylurées de formule (IX)

$$R—NH—CO—NR^1—COCl \qquad (IX)$$

dans laquelle R et $R^1$ ont les définitions indiquées, après quoi, après les avoir éventuellement isolées, on les fait réagir dans une trosième étape avec l'ammoniac, le cas échéant en présence de diluants inertes, à des températures comprises entre 0°C et 100°C.

3. Utilisation des biurets de formule II suivant la revendication 1 pour la préparation de 1,3,5-triazinetriones de formule I

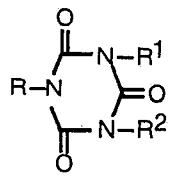

I

dans laquelle R, $R^1$, $R^2$ ont la définition indiquée dans la revendication 1, caractérisée en ce qu'on fait réagir les biurets de formule II avec des esters dialkyliques d'acide carbonique de formule III

$$(R^3O)_2CO \qquad III$$

dans laquelle $R^3$ est un groupe alkyle, en présence de bases fortes, le cas échéant en présence de diluants.

4. N-chlorocarbonylurées de formule (IX)

$$R—NH—CO—NR^1—COCl \qquad (IX)$$

dans laquelle les restes R et $R^1$ possèdent la définition indiquée dans la revendication 1, à l'exclusion de la N-4-(4-chlorophénoxy)-phényl-N'-méthyl-N'-chlorocarbonylurée.

5. Procédé de préparation des N-chlorocarbonylurées de formule IX suivant la revendication 5, caractérisé en ce qu'on fait réagir avec du phosgène des composés de formule VIII

$$R—NH—CO—NR^1H \qquad (VIII)$$

dans laquelle R et $R^1$ ont la définition indiquée dans la revendication 5.

## Claims

1. Biurets of the formula (II)

$$R—NH—CO—NR^1—CO—NHR^2 \qquad (II)$$

in which

R stands for a radical

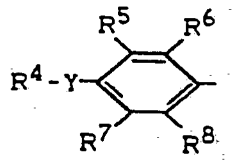

where

$R^4$ stands for phenyl which is optionally substituted by halogen, cyano, nitro, amino, $C_1—C_4$-alkyl, $C_1—C_4$-alkoxy, $C_1—C_4$-alkylthio, $C_1—C_4$-alkylsulphinyl, $C_1—C_4$-alkylsulphonyl, halogeno-$C_1—C_4$-alkyl, halogeno-$C_1—C_4$-alkoxy, halogeno-$C_1—C_4$-alkylthio, halogeno-$C_1—C_4$-alkylsulphonyl, hydroxycarbonyl, $C_1—C_4$-alkylcarbonyl, $C_1—C_4$-alkoxycarbonyl, amiocarbonyl, $C_1—C_4$-alkoxycarbonylamino and/or sulphonylamino or for the radical

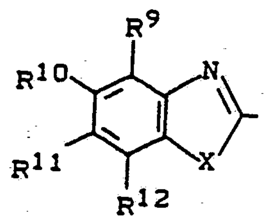

where

$R^9$, $R^{10}$, $R^{11}$ and $R^{12}$ are identical or different and stand for hydrogen, halogen, $C_1—C_4$-alkyl, $C_1—C_4$-alkoxy, halogeno-$C_1—C_4$-alkyl or halogeno-$C_1—C_4$-alkoxy and

X stands for oxygen, sulphur or a grouping —N=CH—,

$R^5$, $R^6$, $R^7$ and $R^8$ are identical or different and stand for hydrogen, halogen, $C_1—C_4$-alkyl or $C_1—C_4$-alkoxy and

Y stands for oxygen, sulphur or the groupings —CO—, —SO—, $—SO_2—$, $—CH_2—$, $—CH_2O—$ or $—OCH_2—$ and

$R^1$ and $R^2$ stand for hydrogen, halogen, $C_1—C_6$-alkyl, $C_1—C_6$-alkoxy, $C_1—C_6$-alkylthio and $C_1—C_6$-alkylsulphonyl, which are optionally substituted by halogen and for phenyl which is optionally substituted by halogen, $C_1—C_2$-alkyl, $C_1—C_2$-alkoxy, $C_1—C_2$-alkylthio, halogeno-$C_1—C_2$-alkyl, halogeno-$C_1—C_2$-alkoxy and/or halogeno-$C_1—C_2$-alkylthio.

2. Process for preparing the biurets of the formula (II)

$$R—NH—CO—NR^1—CO—NHR^2 \qquad (II)$$

in which

R stands for a radical

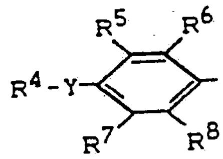

where

$R^4$ stands for phenyl which is optionally substituted by halogen, cyano, nitro, amino, $C_1$—$C_4$-alkyl, $C_1$—$C_4$-alkoxy, $C_1$—$C_4$-alkylthio, $C_1$—$C_4$-alkylsulphinyl, $C_1$—$C_4$-alkylsulphonyl, halogeno-$C_1$—$C_4$-alkyl, halogeno-$C_1$—$C_4$-alkoxy, halogeno-$C_1$—$C_4$-alkylthio, halogeno-$C_1$—$C_4$-alkylsulphonyl, hydroxycarbonyl, $C_1$—$C_4$-alkylcarbonyl, $C_1$—$C_4$-alkoxycarbonyl, aminocarbonyl, $C_1$—$C_4$-alkoxycarbonylamino and/or sulphonylamino or for the radical

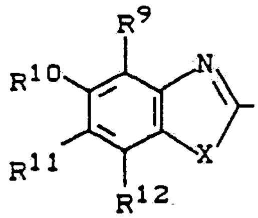

where

$R^9$, $R^{10}$, $R^{11}$ and $R^{12}$ are identical or different and stand for hydrogen, halogen, $C_1$—$C_4$-alkyl, $C_1$—$C_4$-alkoxy, halogeno-$C_1$—$C_4$-alkyl or halogeno-$C_1$—$C_4$-alkoxy and

X stands for oxygen, sulphur or a grouping —N=CH—,

$R^5$, $R^6$, $R^7$ and $R^8$ are identical or different and stand for hydrogen, halogen, $C_1$—$C_4$-alkyl or $C_1$—$C_4$-alkoxy and

Y stands for oxygen, sulphur or the groupings —CO—, —SO—, —$SO_2$—, —$CH_2$—, —$CH_2O$— or —$OCH_2$— and

$R^1$ and $R^2$ stand for hydrogen, halogen, $C_1$—$C_6$-alkyl, $C_1$—$C_6$-alkoxy, $C_1$—$C_6$-alkylthio and $C_1$—$C_6$-alkylsulphonyl, which are optionally substituted by halogen and for phenyl which is optionally substituted by halogen, $C_1$—$C_2$-alkyl, $C_1$—$C_2$-alkoxy, $C_1$—$C_2$-alkylthio, halogeno-$C_1$—$C_2$-alkyl, halogeno-$C_1$—$C_2$-alkoxy and/or halogeno-$C_1$—$C_2$-alkylthio, which is characterized in that

a) isocyanates of the formula (IV)

$$R—NCO \qquad (IV)$$

in which

R has the abovementioned meaning are reacted with ureas of the formula (V)

$$R^1NH—CO—NHR^2 \qquad (V)$$

in which

$R^1$ and $R^2$ have the specified meanings, if desired in the presence of diluents or

b) if compounds of the formula II are prepared in which

$R^2$ stands for hydrogen, characterized in that in the 1st stage amines of the formula (VI)

$$R—NH_2 \qquad (VI)$$

in which R have the abovementioned meanings, are reacted with compounds of the formula (VII)

$$R^1NCO \qquad (VII)$$

in which $R^1$ has the abovementioned meanings, at temperatures between 0°C and 100°C in the presence of inert diluents to give the compounds of the formula (VIII)

$$R—NH—CO—NR^1H \qquad (VIII)$$

in which R and $R^1$ have the abovementioned meanings, and subsequently, if desired after their isolation, are reacted at temperatures between 0°C and 100°C in a 2nd stage with phosphene, if desired in the presence of inert diluents, to give N-chlorocarbonylureas of the formula (IX)

R—NH—CO—$NR^1$—COCl (IX)

in which R and $R^1$ have the abovementioned meanings, and subsequently, if desired after their isolation, are reacted at temperatures between 0°C and 100°C in a 3rd stage with ammonia, if desired in the presence of inert diluents.

3. Use of the biurets of the formula II as set forth in Claim 1 for preparing 1,3,5-triazinetriones of the formula I

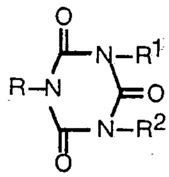

I

in which R, $R^1$ and $R^2$ have the meaning specified in Claim 1, characterized in that the biurets of the formula II are reacted with dialkyl carbonates of the formula III

$(R^3O)_2CO$ III

in which $R^3$ stands for alkyl, in the presence of strong bases and if desired in the presence of diluents.

4. N-Chlorocarbonylureas of the formula (IX)

R—NH—CO—$NR^1$—COCl (IX)

in which the radicals R and $R^1$ have the meaning specified in Claim 1, with the exception of N-4(4-chlorphenoxy)-phenyl-N'-methyl-N'-chlorocarbonylurea.

5. Process for preparing the N-chlorocarbonylureas of the formula IX as set forth in Claim 4, characterized in that the compounds of the formula VIII

R—NH—CO—$NR^1H$ (VIII)

in which R and $R^1$ have the meaning specified in Claim 4, are reacted with phosgene.